Europäisches Patentamt

European Patent Office

Office européen des brevets

(19)

(11) **EP 0 668 999 B1**

(12)

# FASCICULE DE BREVET EUROPEEN

(45) Date de publication et mention
de la délivrance du brevet:
**29.10.1997 Bulletin 1997/44**

(21) Numéro de dépôt: **93910145.7**

(22) Date de dépôt: **19.05.1993**

(51) Int Cl.$^6$: **G01N 33/12**

(86) Numéro de dépôt international:
**PCT/FR93/00496**

(87) Numéro de publication internationale:
**WO 93/24832 (09.12.1993 Gazette 1993/29)**

(54) **APPAREIL DE MESURE DE LA QUALITE DE LA VIANDE**

VORRICHTUNG ZUM MESSEN DER QUALITÄT VON FLEISCH

APPARATUS FOR MEASURING THE QUALITY OF MEAT

(84) Etats contractants désignés:
**BE DE ES FR GB NL PT**

(30) Priorité: **22.05.1992 FR 9206289**

(43) Date de publication de la demande:
**30.08.1995 Bulletin 1995/35**

(73) Titulaire: **SYDEL (S.A.)**
**56100 Lorient (FR)**

(72) Inventeurs:
• **HENRIO, Sylvain**
**F-56440 Languidic (FR)**
• **BERNARD, Luc**
**F-56700 Kervignac (FR)**
• **LEREDDE, Denis**
**F-56100 Lorient (FR)**

(74) Mandataire: **Fournier, Michel Robert Marie et al**
**Cabinet Ballot-Schmit,**
**4 rue Général Hoche**
**56100 Lorient (FR)**

(56) Documents cités:
**EP-A- 0 028 509          EP-A- 0 259 284**
**WO-A-80/01205          WO-A-87/05462**
**WO-A-88/01742          WO-A-92/21025**
**AU-B- 482 112          DE-A- 2 718 561**
**FR-A- 2 334 088          GB-A- 2 179 443**

• **PATENT ABSTRACTS OF JAPAN vol. 14, no. 415**
**(P-1102)(4358) 7 Septembre 1990 & JP-A-21 59**
**542 ( FUJI PHOTO FILM CO LTD ) 19 June 1990**

## Description

La présente invention concerne un appareil de mesure de la qualité de la viande, notamment de la teneur en muscle d'une carcasse.

Les appareils de ce type, tels celui décrit dans le brevet français n° 2 334 088 déposé en 1976 par J.B. HENNESSY, comportent trois éléments principaux : une sonde, un piston de référence de profondeur et un boîtier de mesure. La sonde est une lancette comportant une pointe et une tige équipée d'une tête de mesure, disposée dans une fenêtre proche de la pointe. Cette tête de mesure comprend un photoémetteur et un photorécepteur séparés par une cloison opaque. La lumière émise par l'émetteur est réfléchie et diffusée par le milieu dans lequel est enfoncée la sonde et l'importance de cette réflexion varie en fonction des caractéristiques optiques du milieu traversé. Une brusque diminution du signal délivré par le récepteur indique le passage du gras au muscle, en fonction des coefficients de réflexion différents de ces deux constituants de la carcasse.

Le piston de référence de profondeur est solidaire de deux tiges-supports parallèles coulissantes et il est poussé en avant par un ressort hélicoïdal engagé sur la tige de la sonde. Une de ces tiges-supports entraîne en rotation un cadran indicateur de la profondeur d'enfoncement de la sonde, l'organe d'entraînement étant adapté à débrayer et à se bloquer lorsque le signal de mesure présente une variation brusque caractéristique d'un changement de milieu.

Un inconvénient inhérent à ces appareils de mesure est leur précision insuffisante et leur résolution assez médiocre. En effet, du fait de la taille des composants optoélectroniques utilisés et de leur position relative, les faisceaux émis et reçus sont parallèles et relativement écartés l'un de l'autre. Le signal produit lors d'un changement de milieu ne varie pas d'une façon brutale mais au contraire, progressivement, sur une plage de quelques millimètres.

Un remède à cet inconvénient a été apporté par traitement du signal de mesure comme cela est décrit dans le brevet européen EP-A-0 028 509 également déposé par J.B. HENNESSY. Des valeurs maximale et minimale du signal produit par le photorécepteur qui correspondent respectivement aux intensités de lumière réfléchies par le gras et par le maigre, il est déduit un niveau de signal intermédiaire pris comme référence. Pour les changements de milieu gras/maigre ou inversement, les indications de profondeur sont relevées lorsque le photorécepteur produit un signal égal à cette référence.

Cette modification de l'appareil initial a permis d'améliorer la précision des mesures d'épaisseur de gras et de muscle mais la résolution de ces mesures demeure tout aussi médiocre qu'avant. En effet, du fait que, dans les sondes décrites dans ces brevets, la zone illuminée possède une étendue relativement grande, il est impossible avec ces sondes de détecter la traversée de membranes minces, telles que l'aponévrose, lesquelles, précisément, seraient particulièrement utiles pour la mesure du pourcentage de muscle dans les carcasses de porcs, puisqu'elles entourent la noix de côte.

A cet inconvénient afférent à la mesure des caractéristiques optiques des constituants des carcasses, il faut en ajouter un autre, à savoir, l'indétermination concernant la profondeur d'enfoncement de la lancette dans la carcasse au moment où se produit une transition de milieu. Du fait des mouvements rapides que subit l'appareil au cours d'une mesure et de l'inertie de ses pièces mobiles, la position instantanée du piston de référence de profondeur, pressé avec une force d'appui variable par un simple ressort sur la surface de la carcasse, n'est pas connue avec précision au moment où la transition recherchée se produit. Il s'en suit une erreur de profondeur non négligeable et donc une erreur semblable quant aux pourcentages de gras et/ou de maigre.

L'objet principal de l'invention est un appareil de mesure de la qualité de la viande dont la précision et la résolution de chacune de ces deux mesures concernées (transitions de milieux et profondeur d'enfoncement de la sonde), et de préférence des deux, sont grandement améliorées.

Un objet complémentaire de l'invention est un appareil de mesure de la qualité de la viande, capable en outre de traiter les mesures brutes obtenues afin de produire une grandeur représentative d'un paramètre particulier de cette qualité, le pourcentage de muscle par exemple. Selon une première caractéristique de l'invention, dans un appareil de mesure de la qualité de la viande d'une carcasse, du genre comprenant :

- une sonde en forme de lancette adaptée à pénétrer dans la carcasse, pourvue d'une tête de mesure optique ;
- un appui de référence de profondeur associé à la sonde, destiné à être appliqué sur la carcasse à traiter ;
- un dispositif pour mesurer en permanence la distance séparant la tête de mesure dudit appui ;
- un photoémetteur et un photorécepteur de mesure, constituant la tête de mesure, disposés l'un par rapport à l'autre de manière à produire un signal de mesure analogique, représentatif de la réflectivité lumineuse du milieu environnant ;

est caractérisé en ce que :

- le photoémetteur et le photorécepteur de mesure sont disposés au fond de canaux optiques fins pratiqués dans un support opaque, lesdits canaux étant orientés de manière que leurs axes se croisent en un point de convergence, situé à proximité extérieure immédiate de la surface de la sonde.

En outre, pour permettre de réguler l'intensité de la lumière produite par le photoémetteur, un photorécepteur de référence est prévu et placé dans un troisième canal débouchant dans le canal traversé par la lumière émise par le photoémetteur.

Grâce à ces deux dispositions, la précision, la résolution et la fiabilité d'une sonde optique de mesure de la qualité de la viande, sont grandement améliorées.

Dans une forme de réalisation, l'appareil est un pistolet sur lequel la sonde est montée fixe. L'appui est un piston mobile en translation longitudinale par rapport à la sonde, porté par exemple par deux tiges-supports coulissantes.

Selon une deuxième caractéristique importante de l'invention, les tiges-supports du piston de référence de profondeur entraînent en rotation l'arbre d'un moteur-couple et l'intensité du courant continu qui alimente ce moteur est asservie à égaler une valeur de consigne.

Grâce à cette disposition, les mouvements relativement rapides que l'opérateur fait subir à tout l'appareil au cours d'une mesure n'entraînent pas, malgré l'inertie des masses en mouvement dans l'appareil, des variations indésirables de la force d'appui du piston de référence sur la surface de la carcasse. De telles variations entraînent en effet des enfoncements inégaux du piston dans la carcasse et donc une erreur quant à la profondeur mesurée à un moment donné.

Les caractéristiques et avantages de l'invention apparaîtront d'une manière plus précise à la suite de la description ci-après d'une forme de réalisation, donnée à titre d'exemple non limitatif, en référence aux dessins annexés dans lesquels :

- la figure 1 est une vue schématique du dessus d'un appareil de mesure selon l'invention;
- la figure 2 est une coupe longitudinale à grande échelle du tronçon de sonde dans lequel sont agencés les composants optoélectroniques de mesure;
- la figure 3 est une coupe transversale de ce tronçon de l'appareil;
- la figure 4 est une coupe longitudinale simplifiée de l'appareil;
- la figure 5 est une coupe transversale de l'appareil;
- la figure 6 est le schéma fonctionnel des circuits électroniques de la carte d'acquisition de données de l'appareil;
- la figure 7 est le schéma fonctionnel des circuits électroniques de la carte de gestion de données de l'appareil;
- la figure 8 est le schéma fonctionnel des circuits électroniques d'asservissement de la force d'appui du piston de référence, et
- la figure 9 est une représentation graphique de deux mesures respectivement obtenues au moyen d'un appareil connu et de l'appareil perfectionné selon l'invention.

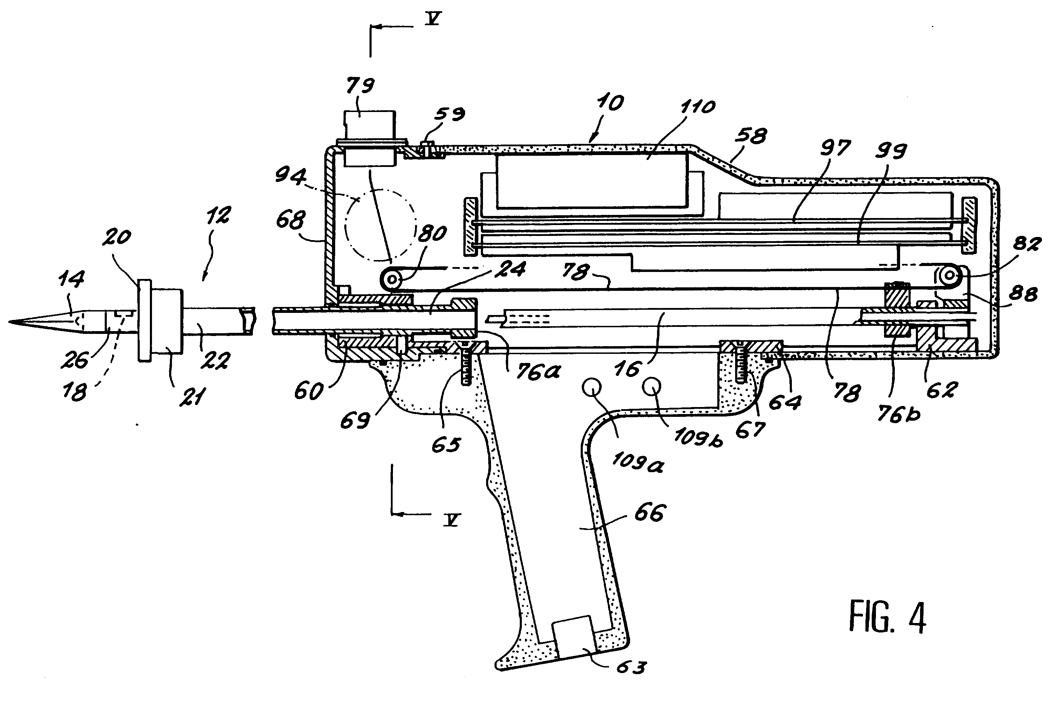

A la figure 4, un appareil de mesure de la qualité de la viande est représenté dans sa forme habituelle, celle d'un pistolet-piqueur muni d'une crosse 66 de préhension.

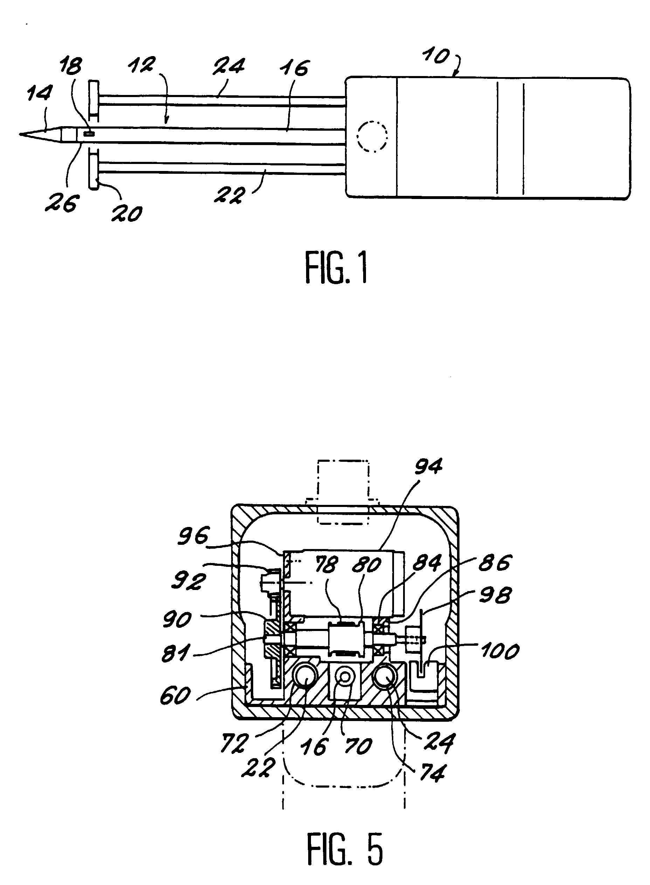

Selon la figure 1, cet appareil comprend un boîtier allongé 10 et une sonde de mesure 12. Cette sonde 12 est constituée par une lancette comportant une pointe acérée 14 et une tige creuse rigide 16, fixée au boîtier 10, une tête de mesure 18 étant aménagée près de la pointe 14. La tige 16 traverse sans contact un piston d'appui 20 monté à l'extrémité de deux tiges-supports 22-24, rigides, parallèles et mobiles longitudinalement à l'intérieur du boîtier 10.

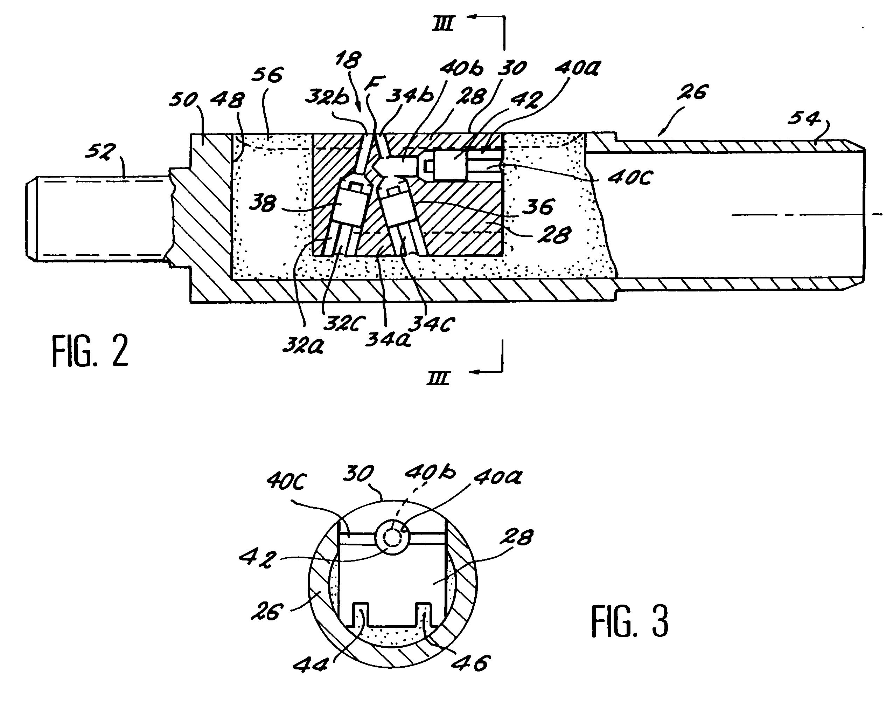

La tête de mesure 18 de la sonde 12 est logée dans un tronçon 26 de la tige creuse 16. Selon les figures 2 et 3 qui représentent respectivement des coupes longitudinale et transversale de ce tronçon 26, la tête de mesure 18 comprend un support 28 en matière plastique opaque, de forme générale parallélépipèdique, avec une face extérieure 30 profilée de manière à prolonger la surface cylindrique du tronçon 26. Le support 28 comporte entre son fond et sa face extérieure 30, deux passages 32-34 dont les axes respectifs convergent à proximité extérieure immédiate de la face extérieure 30.

Les passages 32-34 ont un diamètre élargi dans leurs parties 32a-34a proches du fond du support 28 et un diamètre étroit dans leurs parties 32b-34b débouchant sur la face extérieure 30 du support 28. Dans les parties larges 34a-32a sont respectivement disposés un photoémetteur 36, constitué par exemple par une diode émettrice de rayons rouges et/ou infrarouges, et un photorécepteur 38, constitué par exemple par une photodiode ou un phototransistor sensible à ces rayons.

Les parties étroites 34b-32b des passages 34-32 constituent des canaux fins de guidage de la lumière émise et reçue, qui débouchent côte à côte mais sans se rejoindre dans la face extérieure 30 du support 28. De la sorte, en fonctionnement, il n'y a pas de transmission directe de lumière autour de leur ligne frontière F.

Dans la tête de mesure 18, les deux passages 32-34 sont inclinés longitudinalement d'un même angle voisin de 12°. La valeur de cet angle a été choisie de façon à limiter l'ovalisation des orifices des canaux fins 32b-34b, d'une part, et à faire en sorte que le point de convergence des axes de ces canaux demeure suffisamment proche de la paroi 30 de la tête de mesure, d'autre part. En pratique, les canaux 32b-34b ont avantageusement un diamètre de 0,5 mm, l'écart entre le point de convergence de leurs axes et la paroi de la sonde étant alors de 1 mm environ.

Les diodes photoémettrices sont connues pour être généralement peu stables dans le temps et en température. En conséquence, le support 28 comporte un passage longitudinal 40, à deux diamètres 40a-40b, large et étroit respectivement, qui débouche dans le canal d'émission 34b. Dans la partie large du passage 40 est monté un photorécepteur de référence 42, identique au photorécepteur de mesure 38, destiné à réguler l'intensité de la lumière émise par le photoémetteur 36.

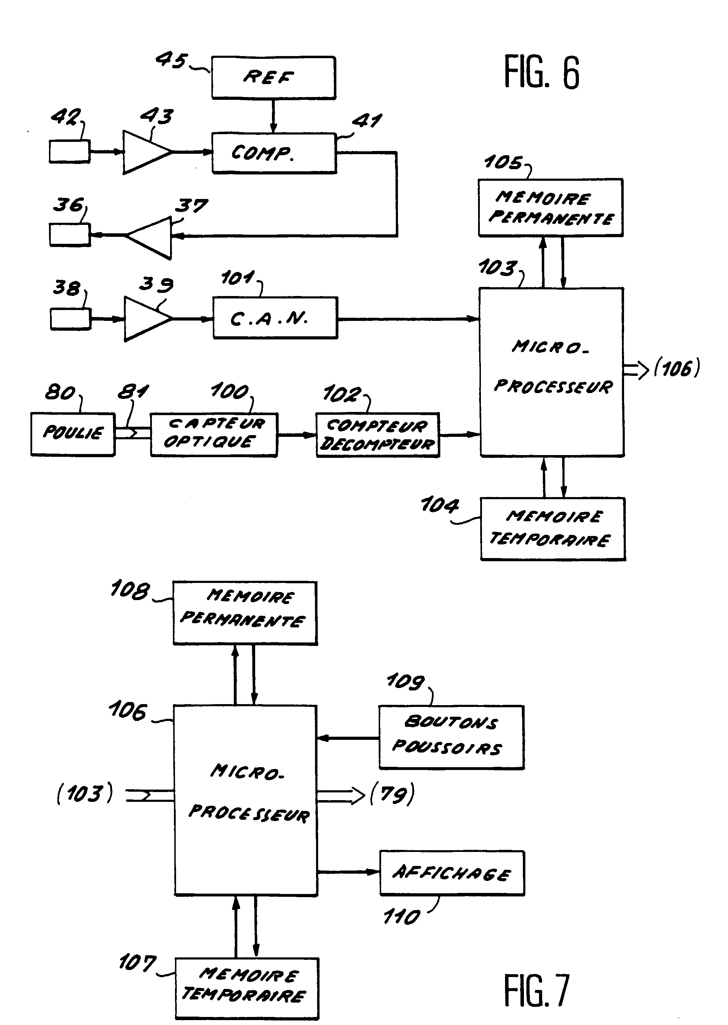

Le circuit de régulation utilisé est représenté schématiquement à la figure 6.

Pour faciliter la mise en place et assurer le maintien des composants électroniques que sont l'émetteur 36 et les récepteurs 38-42, les parties larges 32a, 34a, 40a des passages 32-34-40 comportent des fentes latérales 32c, 34c, 40c. Par ailleurs, deux rainures longitudinales 44-46 sont pratiquées dans le fond du support 28 pour permettre le passage des fils de liaison, vers le boîtier 10, à travers la tige creuse 16 de la sonde 12.

La tête de mesure 18 est installée dans un logement 48 prévu pour elle dans la partie médiane du tronçon 26 de la tige 16 de la sonde 12. L'extrémité 52 du tronçon 26 est un embout plein, fileté et vissé dans la pointe 14, et l'extrémité 54, est un embout creux ajusté et collé dans la tige 16 de la sonde. Le logement 48 débouche sur l'extérieur par une fenêtre longitudinale oblongue à travers laquelle est introduit le support 28 équipé des composants 36-38-42 constituant la tête de mesure 18.

Après insertion, la tête de mesure 18 est scellée au moyen d'une résine 56. Quant aux canaux 32-34-40, ils ont préalablement été obturés au moyen d'une résine transparente dès la mise en place des composants 36-38-42.

Selon la figure 4, la tige creuse et rigide 16 de la sonde 12 est relativement longue et sa partie arrière est interne au boîtier 10 et encastrée dans deux blocs-supports 60-62 constituant les rebords d'un socle 64 du boîtier 10. A ce socle 64 sont fixés par des vis, la crosse 66 du pistolet-piqueur que constitue l'appareil (vis 65-67), une face avant 68 (vis 69) et un capot 58 du boîtier 10. Dans la crosse 66, est monté, un bruiteur 63 constituant une alarme sonore signalant un défaut de mise en oeuvre de l'appareil. Le capot 58 est pincé entre le socle 64 et la crosse 66, d'une part, et fixé par des vis 59 à la face avant 68, d'autre part.

Dans la partie supérieure de la face avant 68 est monté une prise multibroche 79, permettant le raccordement de l'appareil à un calculateur central (non représenté) assurant, une exploitation plus complète des mesures fournies par l'appareil.

Selon la figure 5, le bloc-support 60 et, derrière, la face avant 68, comportent trois passages parallèles : un passage central 70, traversé par la tige fixe 16 de la sonde 12, et des passages latéraux 72-74 traversés par les deux tiges-supports coulissantes parallèles 22-24. Selon la figure 4, les extrémités avant de ces tiges 22-24, sont encastrées dans un bloc 21, sur lequel le piston 20 (une plaque rectangulaire à coins arrondis) est fixé par des vis non représentées.

Selon la figure 5, les extrémités arrières des tiges 22-24, internes au boîtier 10, sont encastrées dans un chariot 76 monté coulissant sur la tige 16 de la sonde 12. Sur la figure 4, ce chariot 76 est représenté schématiquement deux fois, en 76a et 76b, pour figurer les deux positions extrêmes qu'il peut occuper. Le chariot 76 est fixé à une courroie crantée 78, tendue entre deux poulies 80-82 respectivement montées tournantes dans des paliers, tels 84, installés sur des flasques 86 ou 88 solidaires des blocs-supports 60 ou 62. L'arbre de la poulie 82 tourne libre dans ses paliers. L'arbre 81 de la poulie 80 comporte à une extrémité un pignon 90 engrenant avec un pignon plus petit 92, solidaire de l'arbre de sortie d'un moteur-couple à courant continu 94, monté sur une potence 96 prolongeant un des flasques 86.

Sur l'autre extrémité de l'arbre 81, est monté le disque gravé 98 d'un capteur optique de rotation 100, adapté à délivrer un nombre donné relativement élevé (1440 par exemple) d'impulsions, à compter ou à décompter, par tour dans un sens ou dans l'autre de l'arbre 81 de la poulie 80. De la sorte, une impulsion nouvelle est produite pour chaque incrément de déplacement de la courroie 78 (un incrément valant, par exemple, 0,025 mm).

Selon la figure 4, au-dessus de la courroie 78, sont montées deux cartes de circuits imprimés, la carte 97 d'acquisition des données et celle de gestion de ces données, 99. Selon la figure 6, qui représente le schéma fonctionnel général de ces circuits électroniques, le photoémetteur 36 est alimenté par un amplificateur 37 qui reçoit en entrée le signal de sortie d'un comparateur d'amplitudes 41. Les deux entrées de ce comparateur 41 sont respectivement reliées à la sortie d'un amplificateur 43 dont l'entrée est connectée au photorécepteur de référence 42 et à celle d'un potentiomètre de référence 45. Le photorécepteur de mesure 38 est relié à l'entrée d'un amplificateur 39 dont le signal de sortie est appliqué à un convertisseur analogique/numérique 101, du genre à fonctionnement permanent.

De son côté, le capteur optique 100, entraîné par l'arbre 81 de la poulie 80, est relié à un compteur-décompteur 102. Les étages de sortie du convertisseur analogique/numérique 101 et du compteur-décompteur 102 sont raccordés au bus d'entrée d'un premier microprocesseur 103, associé à une première mémoire-temporaire 104, opérant sous le contrôle de logiciels d'exploitation et d'application, contenus dans une première mémoire permanente 105.

Selon la figure 7, qui représente le schéma fonctionnel des circuits électroniques de la carte 99 de gestion de données, le bus de sortie du premier microprocesseur 103 est raccordé au bus d'entrée d'un second microprocesseur 106, associé à une seconde mémoire temporaire 107 et à une seconde mémoire permanente 108 contenant les logiciels d'exploitation et d'application du second microprocesseur 106. Au bus d'entrée du second microprocesseur 106, sont également raccordés des boutons-poussoirs 109 disposés à la partie supérieure de la crosse 66 (voir 109a-109b sur la fig.4). Au bus de sortie du microprocesseur 106 sont connectés d'une part, un dispositif d'affichage à cristaux liquides 110, disposé sous une fenêtre transparente aménagée dans le capot 58 du boîtier 10 (voir fig.4) et, d'autre part, des broches de la prise multibroche 79 fixée sur la face avant 68.

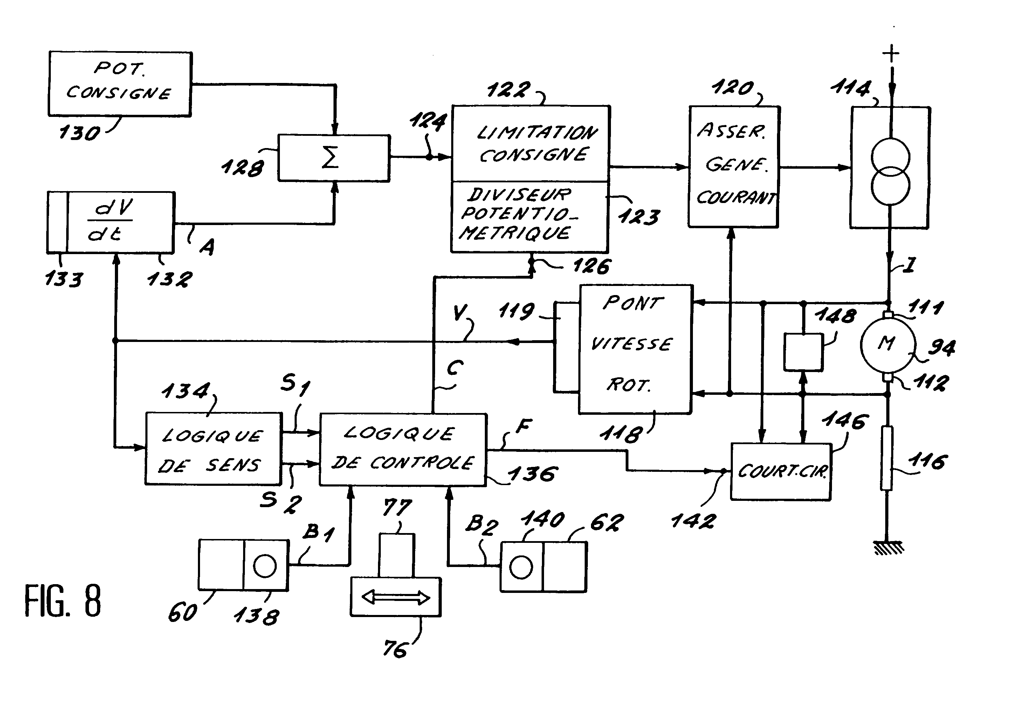

Selon la figure 8, les bornes 111-112 du moteur-couple 94 à courant continu, sont respectivement reliées à la sortie d'un générateur 114 de courant continu I et à une résistance 116, de faible valeur, connectée à la masse. Les bornes 111-112 du moteur 94 sont reliées à un pont de mesure 118, comportant un potentiomètre de réglage 119, adapté à délivrer une tension V de valeur nulle lorsque le moteur 94 est bloqué et de valeurs positive ou négative, proportionnelles aux forces contre-électromotrices développées par le moteur, lorsque celui-ci entraîné par la courroie 78 au cours de la phase de pénétration de la sonde 12 dans une carcasse, tourne dans un premier sens ou lorsque, pendant la phase de retrait de la sonde 12 de la carcasse, il tourne dans l'autre sens. Dans ce deuxième cas la courroie 78, le chariot 76 et le piston 20 ne subissent pas d'efforts extérieurs et le moteur 94 est libre, qui, sous l'action du courant I, tourne jusqu'à ce que le piston 20 soit en butée avant. La borne 112, commune au moteur 94 et à la résistance 116, est reliée à l'une des deux entrées d'un circuit 120 d'asservissement de l'intensité du courant I délivré par le générateur 114, l'autre entrée de ce circuit 120 étant reliée à la sortie d'un circuit 122 de limitation des consignes du courant I, comprenant un diviseur potentiomètrique 123. Le circuit 122 comporte deux types d'entrées : une entrée analogique 124 et une entrée logique 126 adaptée à commander le diviseur 123.

Le signal appliqué à l'entrée analogique 124 est un signal continu qui est fourni par un étage sommateur 128 recevant, d'une part, un signal de consigne pour le courant I, délivré par un potentiomètre 130 et, d'autre part, un signal de correction délivré par un circuit 132. Ce circuit 132 est un étage dérivateur auquel est appliquée la tension V représentative de la vitesse de rotation du moteur 94, fournie par le pont de mesure 118. Le circuit 132, comporte un potentiomètre 133 et délivre un signal analogique A représentatif de l'accélération angulaire du moteur 94, le coefficient de proportionnalité du signal A, étant fixé par le potentiomètre 133.

Le signal V fourni par le pont de mesure 118 est en outre appliqué à l'entrée d'un étage comparateur double 134, adapté à délivrer sur ses deux sorties, deux signaux logiques $S_1$ et $S_2$ respectivement représentatifs des sens de rotation avant et arrière du moteur 94, lorsque la valeur absolue du signal V est supérieure à un seuil Vo. Le signal S1=1 apparaît pendant la phase d'enfoncement de la sonde 12 dans une carcasse et le signal S2=1, pendant la phase de retrait de la sonde. La simultanéité des états $\underline{O}$ des signaux $S_1$ et $S_2$ signifie que le moteur 94 est soit bloqué soit tournant à une vitesse très faible dans un sens ou dans l'autre. Ces signaux $S_1$ et $S_2$ sont appliqués à un circuit logique 136 de contrôle des consignes, recevant par ailleurs deux signaux logiques $B_1$ et $B_2$, représentatifs des butées avant et arrière du chariot 76. Les signaux B1-B2 sont respectivement fournis par deux détecteurs optoélectroniques 138-140, respectivement montés sur les blocs-supports avant et arrière 60-62 de la tige 16 de la sonde 12. Ces détecteurs 138-140 peuvent être occultés par un drapeau 77 relativement large, fixé par son milieu au chariot 76 coulissant sur la tige 16. De la sorte, la simultanéité des états $\underline{O}$ des signaux $B_1$-$B_2$ signifie que le drapeau n'occulte aucun des détecteurs de butée 138-140. Quant à l'état 1 de l'un ou l'autre des signaux $B_1$-$B_2$, il traduit le fait que le drapeau 77 occulte l'un de ces détecteurs de butée. Suivant les valeurs des signaux logiques $S_1$-$S_2$, cela peut signifier, soit que le chariot 76 s'approche ou s'écarte de sa butée physique avant (le support 60) ou arrière (le support 62) si l'un des signaux $S_1$-$S_2$ est égal à 1, soit qu'il est en contact avec l'une de ces butées physiques si les deux signaux $S_1$-$S_2$ sont ensemble à l'état 0. Le circuit logique de contrôle 136 délivre deux signaux logiques C et F respectivement appliqués à l'entrée logique 126 du circuit 122 de limitation de consigne du courant I et à l'entrée logique de commande 142 d'un interrupteur de court-circuit 146 disposé entre les bornes 111-112 du moteur-couple 94. Le diviseur potentiométrique 123, ainsi commandé par le signal C, introduit un facteur de réduction relativement fort (10 par exemple). Les signaux logiques C et F élaborés par l'étage de logique de contrôle 136 répondent aux équations suivantes :

$$C = S_1 + B_1 \text{ et } F = S_1.B_2 + S_2.B_1.$$

Entre les mêmes bornes 111-112 du moteur 94 est également placé un relais électromécanique 148 adapté à être ouvert lorsque l'appareil est en fonctionnement et fermé dans le cas contraire. Le fonctionnement de l'interrupteur 146 et celui du relais 148 ont tous deux pour effet de freiner le piston 20 en faisant un court-circuit aux bornes du moteur 94.

Le pistolet-piqueur représenté à la figure 4 est utilisé soit par un opérateur humain soit par un robot. Dans les deux cas, l'appareil subit des mouvements de translation avant puis arrière et la lancette que constitue la sonde 12 s'enfonce dans la carcasse à mesurer puis s'en retire cependant que le piston de référence de profondeur 20 et les tiges 22-24 qui le portent sont repoussés à l'intérieur du boîtier 10 puis libérés. Les tiges-support 22-24 du piston 20 entraînent le chariot 76 solidaire de la courroie 78, laquelle à son tour, par l'intermédiaire de la poulie 80 et des pignons 90-92, fait tourner le moteur-couple 94. Le disque gravé 98 du capteur optique de rotation 100, entraîné par l'arbre 81 de la poulie 80, délivre des impulsions à compter ou à décompter représentatives des déplacements de la courroie 78. Le compteur-décompteur 102 recevant les impulsions produites par le capteur 100, ayant été préalablement calé à une valeur correspondant à la distance minimale entre la tête de mesure 18 et la face avant du piston 20, (dans ce cas, le chariot 76 est en contact avec le bloc-support 60), le nombre présent à tout moment dans ce compteur-décompteur 102 est représentatif de l'amplitude du déplacement du piston 20 et donc de la profondeur de pénétration de la tête de mesure 18 dans la carcasse concernée.

Pour que cette mesure de profondeur de pénétration de la tête de mesure 18 dans cette carcasse soit correcte, encore faut-il que l'enfoncement de la face avant du piston 20 dans la carcasse soit connue avec précision et soit répétitif d'une mesure à l'autre. L'asservissement à une valeur de consigne de la force d'appui du piston 20 sur une carcasse est assuré par le dispositif décrit à la figure 8.

Selon cette figure 8, le courant I fourni par le générateur 114 qui alimente le moteur-couple 94, est asservi à créer aux bornes de la résistance 116, une tension égale à la valeur de consigne délivrée par l'étage de limitation de consigne 120. En l'absence d'un signal logique C appliqué en commande au diviseur potentiomètrique 123 la valeur de consigne présente en 124, en amont de l'étage 122, se retrouve en aval. Cette valeur de consigne comprend deux termes, l'un principal, fourni par le potentiomètre 130 et l'autre de correction, proportionnel à l'accélération angulaire que subit le moteur 94 et donc à la force appliquée au piston de référence 20 par l'opérateur ou le robot. Le facteur de pondération de ce signal de correction est déterminé d'une manière empirique en fonction des caractéristiques de l'environnement (moteur 94, pont de mesure 118, étage différentiel 132 et tension d'alimentation du potentiomètre 130). En pratique, en fin de fabrication de l'appareil, pour un réglage standard du potentiomètre 130, le gain de l'amplificateur que comporte l'étage 132, est réglé par action sur le potentiomètre 133, de façon que soient compensées les variations, dues à l'inertie des masses en mouvement, de la force d'appui du piston 20 sur une carcasse, qui sont consécutives aux mouvements relativement rapides que l'opérateur fait subir à tout l'appareil. Ces variations de force d'appui sont lues sur un capteur de forces (ressort/potentiomètre), disposé pour le réglage sur la face avant du piston 20.

De la sorte, la force d'appui standard du piston 20 sur les carcasses est tout d'abord déterminée par la valeur maximale du courant de consigne appliqué au moteur-couple 94 lorsque le piston 20, préalablement placé en position arrière, ressort avec la vitesse standard que l'opérateur lui permettra d'avoir pendant la phase de retrait de la sonde 12 de la carcasse à mesurer. Cette valeur maximale de consigne est fixée par le réglage du potentiomètre 130. La force d'appui du piston 20 de chaque appareil sortant de fabrication peut, en conséquence, être aisément réglée à une valeur standard quels que soient les frottements dans les passages 72-74 des tiges 22-24 qui portent ce piston. En outre, grâce au signal de correction de consigne, proportionnel à l'accélération angulaire du moteur 94, les forces d'inertie, dues aux mouvements irréguliers inévitables de l'appareil, sont, après réglage du potentiomètre 133, automatiquement compensées et la force d'appui du piston 20 sur la carcasse est maintenue à sa valeur standard.

Le signal logique $C = S_1 + B_1$, élaboré par l'étage 136, est appliqué en commande à l'entrée 126 du diviseur potentiomètrique 133. Lorsque $B_1 = 1$, le piston 20 est en butée avant, ce qui est le cas depuis le moment où une opération de piquage se termine jusqu'au moment où une autre opération de piquage commence. Dans ce cas, la réduction à sa valeur minimale de la valeur de consigne du courant I alimentant le moteur-couple 94, a uniquement pour objet de soulager la batterie alimentant l'appareil. Lorsque $S_1 = 1$, le piston 20 quitte sa butée avant et la sonde 12 s'enfonce dans la carcasse à mesurer. Dans ce cas, la réduction du courant I a pour objet de diminuer l'effort demandé à l'opérateur pendant la phase de pénétration de la sonde 12 dans la carcasse. En conséquence de ce qui précède, la valeur de consigne du courant I est maximale depuis la fin de la phase de pénétration de la sonde dans la carcasse jusqu'à la fin de la phase de retrait de cette sonde. De la sorte le piston 20 est appliqué sur la carcasse, avec une force d'appui de valeur standard, pendant toute la phase de mesure de la carcasse.

Le signal logique $F = S_1.B_2 + S_2.B_1$, élaboré par l'étage 136, est appliqué en commande à l'interrupteur de court-circuit 146. Lorsque $S_1. B_2 = 1$, le drapeau relativement large 77, solidaire du chariot 76, entraîné par le piston 20, commence à occulter le détecteur photoélectrique 140 de la butée arrière du piston 20. A cet instant, la phase de pénétration de la sonde 12 dans la carcasse est proche de sa fin et, pour éviter un choc du chariot 76 sur la butée mécanique constituée par le bloc-support 62, le moteur 94 est mis en court-circuit par l'interrupteur 146 commandé par le signal logique F.

Ce qui a pour effet de freiner considérablement la rotation du moteur 94 et le mouvement de recul du chariot 76. Dès que la vitesse, de rotation du moteur 94 tombe en dessous de la valeur de seuil Vo, le signal $S_1 = 0$ et donc $F = 0$. Ce qui a pour effet de libérer le moteur 94 afin de lui permettre d'appliquer au piston 20 une force d'appui standard, dès le début de la phase de retrait de la sonde.

Lorsque le drapeau 77 commence à occulter le détecteur photoélectrique 138 de la butée avant du piston 20, la phase de retrait de la sonde est proche de sa fin et $F = S_2.B_1 = 1$. Ce qui a pour effet de court-circuiter et freiner le moteur 94 jusqu'à ce que démarre une autre opération de piquage. En outre, pendant cet intervalle entre deux opérations de piquage successives, la valeur de consigne du courant I circulant dans l'interrupteur 146 court-circuitant le moteur 94, est minimale.

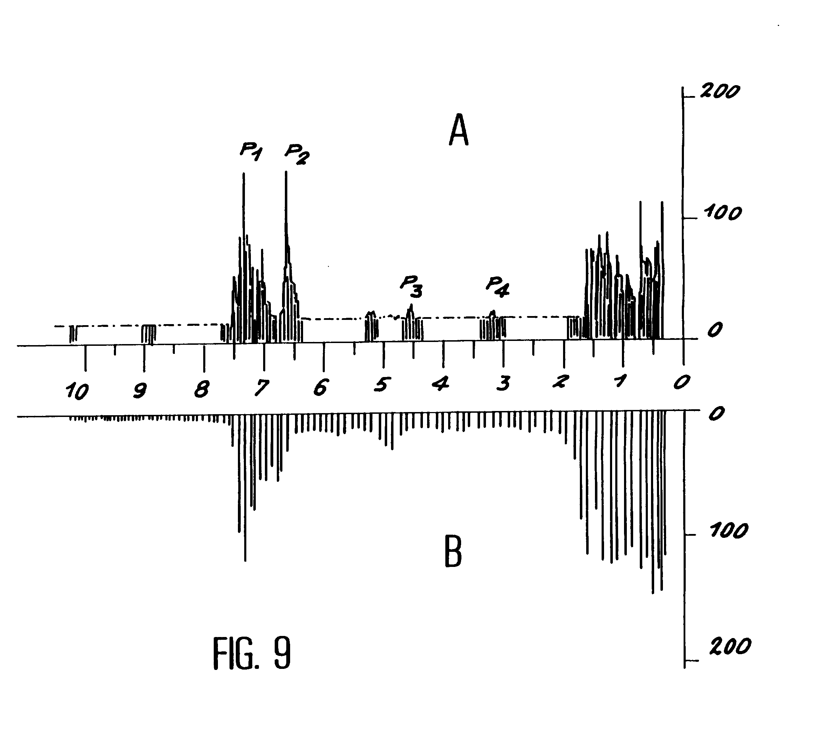

La figure 9 est une représentation des résultats des deux mesures respectivement effectuées sur une même carcasse de porc au moyen d'un appareil perfectionné selon l'invention (graphique A) et d'un appareil de type connu (graphique B). Les profondeurs d'enfoncement de la sonde sont indiquées en abscisses et les valeurs des mesures de réflectivité lumineuse des couches traversées, en ordonnées. La sonde est, successivement enfoncée en deux points particuliers de la carcasse, situés entre deux paires de côtes, jusqu'à ce qu'elles ressortent dans le vide thoracique. Les mesures étant réalisées lors du retrait de la sonde, les signaux sont enregistrés de la gauche vers la droite sur les graphiques. Tout d'abord chacun des signaux est minimal et révèle le vide thoracique. Puis leur croissance, à partir de la cote 7,6 cm traduit le passage dans une zone graisseuse ou cartilagineuse. Si l'on compare les graphiques A et B dans la couche allant des cotes 7,6 à 6,4 cm, on voit tout d'abord que le nombre de mesures effectuées dans le premier cas est très supérieur à celui du second. La résolution des mesures de profondeur de l'appareil perfectionné selon l'invention est très supérieure à celle des appareils de type connu. De plus si l'on compare deux mesures successives effectuées en un même site avec l'appareil selon l'invention puis avec un appareil de type connu, on constate, dans le premier cas, une excellente répétabilité des résultats et, dans le second, une dispersion non négligeable. Cela est dû dans le premier cas, au fait que le piston 20 est appliqué sur la carcasse avec une force d'appui constante au cours des deux opérations et, dans le second cas, au fait que cette force d'appui ne peut être constante, quelle que soit l'attention apportée à cet effet par l'opérateur. Avec un appareil selon l'invention les mesures de profondeur présentent une résolution, une précision et une fiabilité particulièrement grandes.

Ensuite, on voit qu'une mesure particulière peut prendre une amplitude très grande par rapport à celle de la mesure qui la précède ou qui la suit immédiatement, ce qui n'est pas le cas des mesures du graphique B. Cela est dû à la très grande résolution des mesures de réflectivité lumineuse apportée par la tête de mesure de la sonde selon l'invention : canaux optiques fins 32b-34b, convergeant à proximité immédiate de la paroi de la tête 18.

Cette double résolution améliorée des mesures fournies par l'appareil selon l'invention permet, dans la couche définie par les côtes 7,6 => 6,4 cm, de repérer en plus du pic $P_1$, commun aux deux graphiques, (il caractérise un cartilage) un deuxième pic $P_2$ particulièrement important qui n'apparaît pas sur le graphique B. Ce pic $P_2$ correspond à l'aponévrose, membrane de quelques dixièmes de millimètre d'épaisseur, qui entoure la noix de côte. C'est à partir de la position du pic $P_2$ que commence le muscle, lequel correspond au passage de la côte 6,6 à la côte 1,6. Le niveau du signal de mesure dans cette traversée est stable sauf en deux points particuliers repérés par les pics $P_3$-$P_4$ qui indiquent la présence, dans la masse du muscle, de couches de tissus graisseux relativement minces. La sonde de type connu n'a pas pu détecter ces couches minces du fait de sa résolution insuffisante. A partir de la côte 1,6 cm, le passage du maigre au gras de surface sur le graphique A, apparaît d'une manière beaucoup plus nette que sur le graphique B.

D'une manière générale, la double résolution (mesure de la réflectibilité lumineuse des couches traversées et mesure de la profondeur de ces couches) de l'appareil perfectionné selon l'invention est environ dix fois supérieure à la double résolution des appareils de type connu. Dans ces conditions, l'appareil selon l'invention apporte des possibilités nouvelles à l'exploitation des données fournies. La précision de ces données permet en effet d'utiliser l'appareil de mesure de la qualité de la viande selon l'invention pour des applications nouvelles et notamment pour des mesures sur des viandes autres que porcines, telles que les viandes ovines ou bovines (mesure du persillage).

Une opération complète de mesure du pourcentage de muscle d'une demi-carcasse de porc comprend deux piquages successifs.

Les deux sites de ces piquages sont imposés par les services administratifs officiels concernés. Le premier site est situé entre la troisième et la quatrième vertèbre lombaire, à huit centimètres de la fente d'une demi-carcasse. Le second site est situé entre la troisième et la quatrième sous-dernière côte, à six centimètres de la fente.

Au fur et à mesure qu'un piquage est effectué le microprocesseur 103 de l'étage d'acquisition de données, opérant sous le contrôle du premier logiciel d'application, réalise successivement les opérations suivantes :

(1) appeler périodiquement (à une fréquence de 10 KHz par exemple) les contenus des étages de sortie du convertisseur analogique-numérique 101 et du compteur-décompteur 102, pendant la phase de retrait de la sonde 12;
(2) combiner les deux nombres présents à cet instant dans ces étages de sortie, pour en faire deux champs associés de bits de mesure;
(3) appliquer ces champs de bits à la mémoire temporaire 104 pour progressivement constituer une trame de données brutes, correspondant aux mesures simultanées de réflectivité lumineuse et de profondeur de sonde, effectuées au cours d'une opération de piquage;
(4) transférer cette trame de données brutes au microprocesseur 106 de l'étage de gestion des données, à la fin de chaque opération de piquage.

Le microprocesseur 106 opérant sous le contrôle du second logiciel d'application, est adapté à réaliser les opérations ci-après :

(1) en réponse aux instructions de l'opérateur, utilisant à cet effet les boutons-poussoirs 109, afficher sur le dispositif d'affichage 110 le menu des différentes fonctions possibles de l'étage de gestion des données;
(2) en réponse aux instructions de l'opérateur, mettre à jour le numéro de la carcasse à traiter et stocker ce numéro dans la mémoire temporaire 107;
(3) stocker dans la mémoire temporaire 107, la trame de données brutes transférée par le microprocesseur 103 de l'étage d'acquisition de données;
(4) analyser la trame de données brutes ainsi stockée, pour y détecter les points caractéristiques à retenir pour le calcul de la valeur du paramètre de qualité de viande concerné;

(5) stocker les profondeurs de ces points caractéristiques dans la mémoire temporaire 107;

(6) commander le fonctionnement d'une alarme 63 au cas où les points caractéristiques en question n'auraient pas pu être détectés dans la trame de données brutes analysées, ladite alarme informant l'opérateur que l'opération de piquage effectuée n'est pas correcte et doit être recommencée;

(7) combiner les profondeurs des points caractéristiques détectés dans deux trames, de données brutes relevées sur une même carcasse, selon une équation conforme à la réglementation applicable au paramètre de qualité recherché, afférent au type de carcasses mesurées, afin de produire une valeur numérique représentative de ce paramètre;

(8) stocker cette valeur numérique dans la mémoire temporaire 107;

(9) afficher la dernière valeur numérique ainsi calculée sur le dispositif d'affichage 110;

(10) incrémenter d'une unité le numéro de carcasse correspondant à la prochaine trame de données brutes à recevoir de l'étage d'acquisition de données;

(11) en réponse à une instruction de l'opérateur, transférer à un calculateur central le contenu de la mémoire temporaire 107.

En pratique la mémoire temporaire 107 de l'étage de gestion des données pourra contenir une centaine de trames de données brutes correspondant à une cinquantaine de carcasses mesurées ainsi que les profondeurs des points caractéristiques détectés dans ces trames et les valeurs numériques du paramètre de qualité calculées à partir de ces points. En conséquence, l'opérateur pourra procéder à la mesure d'environ cinquante carcasses avant de transférer à un calculateur central l'ensemble des données recueillies et des valeurs calculées par l'appareil de mesure selon l'invention.

Lorsque l'appareil de mesure selon l'invention sera utilisé pour des carcasses autres que de porc, (boeuf ou mouton notamment) les fonctions de l'étage d'acquisition de données seront pratiquement inchangées, même si la procédure suivie par l'opérateur au cours d'un piquage est notablement différente de celle mise en oeuvre pour des carcasses de porc. En revanche, les opérations (4) et (7) réalisées par le microprocesseur 106 de l'étage de gestion des données seront bien entendu, différentes de celles prévues pour une carcasse de porc. Pour l'opération (4), les points caractéristiques à retenir seront des points spécifiques du type de carcasses à traiter. De même pour l'opération (7) l'équation à utiliser sera propre à ces carcasses.

A titre de variante on notera que l'appareil de mesure selon l'invention peut ne comporter qu'un étage d'acquisition de données et que, dans le cas où l'appareil est constamment connecté à un calculateur central, cet étage pourra ne réaliser que les opérations (1) et (2) visées plus haut, les opérations (3) et (4) décrites étant remplacées par la suivante :

- appliquer ces deux champs associés à une mémoire temporaire d'un calculateur central pour constituer progressivement une trame de données brutes correspondant aux mesures simultanées de réflectivité lumineuse et de profondeur de sonde, effectuées au cours d'une opération de piquage.

Dans un tel cas, les opérations (3) et (4) initialement réalisées par l'étage d'acquisition des données et l'ensemble des opérations réalisées par l'étage de gestion de données, seront prises en charge par le calculateur central.

Cette description n'est évidemment pas limitative.

Notamment, bien qu'elle fasse état d'une sonde montée fixe sur un boîtier, associée à un appui de référence de profondeur mobile, il va de soi qu'une combinaison inverse est parfaitement envisageable, soit une sonde mobile, associée à un appui fixe.

De même, en ce qui concerne les moyens lumineux (émission, détection et régulation), ils ont été présentés comme étant dans l'extrémité de détection de la sonde, mais ils pourraient aussi bien se trouver en un emplacement distant, ce qui nécessiterait seulement de prolonger vers l'intérieur les canaux de transfert de la lumière jusqu'à cet emplacement distant.

## Revendications

1.   Appareil de mesure de la qualité de la viande d'une carcasse du genre, comprenant :

   - une sonde (12) en forme de lancette adaptée à pénétrer dans la carcasse, pourvue d'une tête de mesure optique (18) ;
   - un appui de référence de profondeur (20) associé à la sonde (12), destiné à être appliqué sur la carcasse à traiter ;
   - un dispositif (78-100) pour mesurer en permanence la distance séparant la tête de mesure (18) dudit appui (20)
   - un photoémetteur (36) et un photorécepteur de mesure (38), constituant la tête de mesure (18), disposés l'un par rapport à l'autre de manière à produire un signal de mesure analogique, représentatif de la réflectivité lumineuse du milieu environnant ;
   - caractérisé en ce que :
   - le photoémetteur (36) et le photorécepteur de mesure (38) sont disposés au fond de canaux optiques fins (34b-32b) pratiqués dans un support opaque (28), lesdits canaux étant orientés de manière que leurs axes se croisent en un point de convergence, situé à proximité extérieure immmédiate de la surface de la sonde (12).

**2.** Appareil de mesure selon la revendication 1, caractérisé en ce qu'un photorécepteur de régulation d'émission (42) est disposé au fond d'un canal optique (40b) débouchant dans le canal fin (34b) du photoémetteur (36).

**3.** Appareil de mesure selon la revendication 1 ou 2, caractérisé en ce que les canaux optiques (34b-32b) d'émission et de réception sont symétriquement inclinés d'un angle de 12° environ par rapport à un plan transversal de la tige (16) de la sonde (12).

**4.** Appareil de mesure selon l'une des revendications précédentes, caractérisé en ce que les canaux optiques (32b-34b) ont un diamètre de 0,5 mm environ et sont remplis d'une résine transparente.

**5.** Appareil de mesure de la qualité de la viande d'une carcasse selon l'une des revendications précédentes, caractérisé en ce que les moyens pour appliquer l'appui (20) sur la carcasse à traiter comprennent un moteur-couple (94) asservi à délivrer un couple maintenant à une valeur de consigne la force d'application de l'appui (20) sur la carcasse.

**6.** Appareil de mesure selon la revendication 5 du genre dans lequel la sonde (12) est une tige creuse rigide (16) montée fixe dans un boîtier (10), et l'appui (20), un piston mobile, solidaire de deux tiges-supports (22-24) adaptées à coulisser dans le boîtier (10) et à entraîner le dispositif de mesure de la profondeur d'enfoncement de la tête de mesure (18), caractérisé en ce que la tige (16) de la sonde (12) est, dans sa partie interne au boîtier (10), encastrée dans deux blocs-supports (60-62) respectivement fixés aux extrémités du socle (64) dudit boîtier, les tiges-supports (22-24) du piston (20) sont montées coulissantes dans le support avant (60), et encastrées dans un chariot (76) monté coulissant sur ladite partie de la tige (16) interne au boîtier (10), ledit chariot (76) est solidaire d'une courroie crantée (78) tendue entre deux poulies (80-82) respectivement montées tournantes dans des paliers portés par lesdits blocs-supports (60-62), l'arbre (81) de la poulie (80) comportant, à une extrémité, un capteur optique de rotation angulaire (98-100) à haute résolution, délivrant des impulsions à compter où à décompter en fonction du sens de la rotation et, à l'autre extrémité, un premier pignon (90) engrenant avec un second pignon (92), fixé à l'arbre (81) du moteur-couple (94).

**7.** Appareil de mesure selon la revendication 5 du genre dans lequel la sonde (12) est une tige creuse rigide (16) montée fixe dans un boîtier (10), et l'appui (20), un piston mobile, solidaire de deux tiges-supports (22-24) adaptées à coulisser dans le boîtier (10) et à entraîner le dispositif de mesure de la profondeur d'enfoncement de la tête de mesure (18), caractérisé en ce que des moyens (116-118-119) sont prévus pour produire un signal analogique représentatif de la vitesse de rotation du moteur (94), en ce qu'un étage dérivateur (132), pourvu d'un potentiomètre de réglage de gain (133), est adapté à délivrer un signal analogique représentatif de l'accélération angulaire du moteur (94) et en ce qu'un étage de sommation (128) est adapté à additionner un signal de consigne fourni par un potentiomètre (130) et ledit signal d'accélération, le réglage du potentiomètre de gain (133) étant choisi de façon que soient compensées les variations de la force d'appui du piston (20) sur une carcasse, dues à l'inertie des masses mobiles de l'appareil au cours d'une opération de mesure.

**8.** Appareil de mesure selon les revendications 6 et 7, caractérisé en ce que :

- le signal de vitesse de rotation du moteur (94) est appliqué à l'entrée d'un étage de détection du sens de rotation (134), adapté à délivrer deux signaux logiques $S_1$-$S_2$ représentatifs de ces sens, lorsque la valeur absolue de cette vitesse est supérieure à un seuil donné;

- un drapeau (77), relativement large, porté par le chariot (76) solidaire des tiges-supports (22-24) du piston (20), est adapté à occulter deux détecteurs optiques de butée (138-140) respectivement montés sur les blocs-supports (60-62) de la tige (16) de la sonde (12) et adaptés à délivrer deux signaux logiques $B_1$-$B_2$;

- un circuit logique (136) adapté à recevoir les signaux $S_1$-$S_2$ et $B_1$-$B_2$ et à les combiner pour produire deux signaux logiques $C = S_1 + B_1$ et $F = S_1 . B_2 + S_2 . B_1$;

- ledit signal logique C étant appliqué en commande à un diviseur potentiomètrique (123) disposé en série dans la liaison établie entre l'étage de sommation (128) et l'étage d'asservissement de courant (120).

- ledit signal logique F étant appliqué en commande de fermeture à un interrupteur (146) disposé en court-circuit entre les bornes du moteur (94).

**9.** Appareil de mesure selon les revendications 2 et 6, caractérisé en ce qu'il comporte :

- des circuits électroniques (37-41-43-45) pour asservir à une valeur de consigne la quantité de lumière, émise par le photoémetteur (36), reçue par le photorécepteur de régulation (42);

- un convertisseur analogique/numérique (101) pour délivrer un signal numérique représentatif de la lumière reçue par le photorécepteur de mesure (38) et de la réflectivité des couches traversées par la sonde (12);

- un étage compteur-décompteur (102) pour compter et décompter en fonction du sens de rotation du cadran gradué (98) d'un capteur optique de rotation (100), les impulsions délivrées par ce capteur et, par là, constamment fournir la profondeur d'enfoncement de la sonde (12) dans la carcasse;
- un étage d'acquisition de données comprenant un microprocesseur (103), une mémoire temporaire (104) et une mémoire permanente (105) contenant un premier logiciel d'exploitation et un premier logiciel d'application;
- ledit microprocesseur 103 opérant sous le contrôle dudit premier logiciel d'application étant adapté à :

    (1) appeler périodiquement les contenus des étages de sortie du convertisseur analogique-numérique (101) et du compteur-décompteur (102), pendant la phase de retrait de la sonde (12);

    (2) combiner les deux nombres présents à cet instant dans ces étages de sortie, pour en faire deux champs associés de bits de mesure;

    (3) appliquer ces champs de bits à une mémoire temporaire (104) pour constituer progressivement une trame de données brutes, correspondant aux mesures simultanées de réflectivité lumineuse et de profondeur de sonde, effectuées au cours d'une opération de piquage.

10. Appareil de mesure selon la revendication 9, caractérisé en ce que :

- le microprocesseur (103) de l'étage d'acquisition de données opérant sous le contrôle du premier logiciel d'application est adapté, à la fin de chaque opération de piquage, à transférer chaque trame de données brutes à un étage de gestion de données;
- ledit étage de gestion de données comprenant un microprocesseur (106), une mémoire temporaire (107) et une mémoire permanente (108) contenant un second logiciel d'exploitation et un second logiciel d'application, des boutons-poussoirs (109) et un dispositif d'affichage (110) pour établir une liaison interactive entre ledit étage de gestion de données et l'opérateur;
- ledit microprocesseur (106) opérant sous le contrôle du second logiciel d'application est adapté à réaliser les opérations suivantes :

    (1) en réponse aux instructions de l'opérateur, utilisant à cet effet les boutons-poussoirs (109), afficher sur le dispositif d'affichage (110) le menu des différentes fonctions possibles de l'étage de gestion des données;

(2) en réponse aux instructions de l'opérateur, mettre à jour le numéro de la carcasse à traiter et stocker ce numéro dans la mémoire temporaire (107);

(3) stocker dans la mémoire temporaire (107), la trame de données brutes transférée par le microprocesseur (103) de l'étage d'acquisition de données;

(4) analyser la trame de données brutes ainsi stockée, pour y détecter les points caractéristiques à retenir pour le calcul de la valeur du paramètre de qualité de viande concerné;

(5) stocker les profondeurs de ces points caractéristiques dans la mémoire temporaire (107);

(6) commander le fonctionnement d'une alarme (63) au cas où les points caractéristiques en question n'auraient pas pu être détectés dans la trame de données brutes analysées, ladite alarme informant l'opérateur que l'opération de piquage effectuée n'est pas correcte et doit être recommencée;

(7) combiner les profondeurs des points caractéristiques détectés dans deux trames de données brutes relevées sur une même carcasse, selon une équation conforme à la réglementation applicable au paramètre de qualité recherché, afférent au type de carcasses mesurées, afin de produire une valeur numérique représentative de ce paramètre;

(8) stocker cette valeur numérique dans la mémoire temporaire (107);

(9) afficher la dernière valeur numérique ainsi calculée sur le dispositif d'affichage (110);

(10) incrémenter d'une unité le numéro de carcasse correspondant à la prochaine trame de données brutes à recevoir de l'étage d'acquisition de données;

(11) en réponse à une instruction de l'opérateur, transférer à un calculateur central le contenu de la mémoire temporaire (107).

**Patentansprüche**

1. Vorrichtung zum Messen der Qualität von Fleisch eines typischen Fleischstücks, umfassend:

- eine Sonde (12) in Form einer zum Eindringen in das Fleischstück geeigneten Lanzette, versehen mit einem optischen Meßkopf (18);

- einen mit der Sonde (12) verbundenen Tiefenbezugsansatz (20), der dafür vorgesehen ist, an das zu behandelnde Fleischstück angesetzt zu werden;
- eine Vorrichtung (78-100) zum ständigen Messen des Abstands des Meßkopfs (18) vom Absatz (20);
- einen Photosender (36) und einen Meßphotoempfänger (38), die den Meßkopf (18) bilden, und die so zueinander angeordnet sind, daß sie ein analoges Meßsignal erzeugen, das die Reflexion des Lichtes der Umgebung darstellt;

dadurch gekennzeichnet, daß:
der Photosender (36) und der Meßphotoempfänger (38) am Ende von feinen optischen, in einem lichtundurchlässigen Träger angelegten Kanälen (34b-32b) angeordnet sind, wobei die Kanäle so ausgerichtet sind, daß ihre Achsen sich in einem Konvergenzpunkt kreuzen, der in direkter äußerer Nähe der Oberfläche der Sonde (12) liegt.

2. Meßvorrichtung nach Anspruch 1, dadurch gekennzeichnet, daß ein Photoempfänger zur Regelung der Emission (42) am Ende eines optischen Kanals (40b) angeordnet ist, der in den dünnen Kanal (34b) des Photosenders (36) mündet.

3. Meßvorrichtung nach Anspruch 1 oder 2, dadurch gekennzeichnet, daß die optischen Sende- und Empfangskanäle (34b-32b) symmetrisch um einen Winkel von ca. 12° gegenüber einer quer zum Schaft (16) der Sonde (12) verlaufenden Ebene geneigt sind.

4. Meßvorrichtung nach einem der vorhergehenden Ansprüche, dadurch gekennzeichnet, daß die optischen Kanäle (32b-34b) einen Durchmesser von ca. 0,5 mm haben und mit einem durchsichtigen Harz gefüllt sind.

5. Vorrichtung zum Messen der Qualität von Fleisch eines Fleischstücks nach einem der vorhergehenden Ansprüche, dadurch gekennzeichnet, daß die Einrichtung zum Ansetzen des Ansatzes (20) auf das zu behandelnde Fleischstück einen Drehmomentmotor (94) aufweist, der dazu dient, ein Drehmoment zu liefern, das die Ansatzkraft des Ansatzes (20) auf das Fleischstück auf einem Einstellwert hält.

6. Meßvorrichtung nach Anspruch 5, in der die Sonde (12) ein fest in einem Gehäuse (10) angebrachter starrer hohler Schaft (12) und der Ansatz (20) ein beweglicher Kolben ist, der fest mit zwei Träger-Stangen (22-24) verbunden ist, die geeignet sind, in dem Gehäuse (10) zu gleiten und die Vorrichtung zum Messen der Eindringtiefe des Meßkopfes (18) anzutreiben, dadurch gekennzeichnet, daß der Schaft der Sonde (12) in ihrem im Gehäuse (10) liegenden Teil in zwei jeweils an den Enden der Grundplatte (64) des Gehäuses befestigten Trägerblöcken (60-62) eingebaut ist, die Träger-Stangen (22-24) des Kolbens (20) verschiebbar in dem vorderen Träger (60) angebracht und in einem Wagen (76) eingebaut sind, der gleitend auf dem genannten im Inneren des Gehäuses (10) liegenden Teil des Schaftes angebracht ist, der Wagen (76) mit einem Zahnriemen (78) verbunden ist, der zwischen zwei Riemenscheiben (80-82) gespannt ist, die jeweils drehend in von den Trägerblöcken getragenen Lagern (60-62) angeordnet sind, wobei die Achse (81) der Riemenscheibe (80) an einem Ende einen optischen Drehwinkelgeber (98-100) mit hoher Auflösung, der Impulse abgibt, die in Abhängigkeit von der Drehrichtung zu addieren oder zu subtrahieren sind, und am anderen Ende ein erstes Ritzel (90) aufweist, das mit einem zweiten Ritzel (92) in Eingriff ist, das an der Achse (81) des Drehmomentmotors (94) befestigt ist.

7. Meßvorrichtung nach Anspruch 5, in der die Sonde (12) ein fest in einem Gehäuse (10) angebrachter starrer hohler Schaft (12) und das Auflager (20) ein beweglicher Kolben ist, der mit zwei Träger-Stangen (22-24) verbunden ist, die geeignet sind, in dem Gehäuse (10) zu gleiten und die Vorrichtung zum Messen der Eindringtiefe des Meßkopfes (18) anzutreiben, dadurch gekennzeichnet, daß Einrichtungen (116-118-119) vorgesehen sind, um ein analoges Signal zu erzeugen, das die Drehzahl des Motors (94) darstellt, daß eine mit einem Verstärkungsregelungspotentiometer (133) versehene Differenziererstufe (132) ausgelegt ist, ein analoges Signal zu erzeugen, das die Winkelbeschleunigung des Motors (94) darstellt, und daß eine Summierungsstufe (128) dazu ausgelegt ist, ein von einem Potentiometer (130) geliefertes Einstellsignal und das Beschleunigungssignal zu addieren, wobei die Verstärkungsregelung (133) des Potentiometers so gewählt wird, daß die durch die Trägheit der beweglichen Teile des Gerätes während eines Meßvorgangs bedingten Schwankungen der Ansatzkraft des Kolbens (20) auf ein Fleischstück ausgeglichen werden.

8. Meßvorrichtung nach Anspruch 7 und 8, dadurch gekennzeichnet, daß:

- das Motordrehzahl-Signal (94) an dem Eingang einer Erfassungsstufe der Drehrichtung (134) angelegt wird, die dazu ausgelegt ist, zwei logische, diese Richtungen darstellende Signale S1-S2 abzugeben, wenn der absolute Wert dieser Drehzahl höher als ein gegebener Schwellenwert ist;

- eine relativ große Markierung (77) von dem fest mit den Träger-Stangen (22-24) des Kolbens (20) verbundenen Wagen getragen wird und dazu ausgelegt ist, zwei optische Anschlagsdetektoren (138-140) zu verdunkeln, die jeweils auf den Träger-Blöcken (60-62) des Schafts (16) der Sonde (12) befestigt sind und dazu ausgelegt sind, zwei logische Signale B1-B2 abzugeben;

- eine logische Schaltung (136) dazu ausgelegt ist, die Signale S1-S2 und B1-B2 aufzunehmen und sie zu kombinieren, um zwei logische Signale C = S1 + B1 und F = S1 . B2 + S2 . B1 zu erzeugen;

- das logische Signal C bei Bedarf an einem potentiometrischen Teiler (123) angelegt wird, der in der zwischen der Summierungsstufe (128) und der Stromlieferstufe (120) hergestellten Verbindung in Reihe angeordnet ist;

- das logische Signal F bei Schließbedarf an einem Schalter (146) angelegt wird, der zwischen den Klemmen des Motors (94) kurzschlußmäßig angeordnet ist.

9. Meßvorrichtung nach den Ansprüchen 2 und 6, dadurch gekennzeichnet, daß sie aufweist:

- elektronische Schaltungen (37-41-43-45), um die Lichtmenge auf einen Einstellwert zu regeln, die von dem Photosender (36) abgegeben und von dem Regelungs-Photoempfänger (42) aufgenommen wird;

- einen Analog-Digital-Umsetzer (101), um ein numerisches Signal abzugeben, das das vom Meßphotoempfänger (38) aufgenommene Licht und die Reflektivität der von der Sonde durchquerten Schichten darstellt;

- eine Addier-Subtrahier-Stufe (102), um in Abhängigkeit von der Drehrichtung der Skalenscheibe (98) eines optischen Drehsensors (100) die von diesem Sensor gelieferten Impulse zu addieren und zu subtrahieren und dadurch ständig die Eindringtiefe der Sonde (12) in das Fleischstück zu liefern,

- eine Datenaufnahmestufe mit einem Mikroprozessor (103), einem temporären Speicher (104) und einem permanenten Speicher (105), ein erstes Auswerteprogramm und ein erstes Anwendungsprogramm enthaltend;

  - wobei der Mikroprozessor (103) gesteuert wird durch das erste Anwendungsprogramm und ausgelegt ist, um:

    (1) periodisch die Inhalte der Ausgangsstufen des Analog-Digital-Umsetzers (101) und des Addierer-Subtrahierers während der Phase des Herausziehens der Sonde (12) abzufragen;

    (2) die zwei Ziffern zu kombinieren, die zu diesem Zeitpunkt an den Ausgangsstufen anliegen, um daraus zwei zusammenhängende Meßbitgrößen zu machen;

    (3) diese Bitgrößen an einem temporären Speicher (104) anzulegen, um nach und nach eine Rohdatenbasis zu schaffen, die den simultanen Messungen der Lichtreflexion und der Tiefe der Sonde entsprechen, die im Verlauf eines Einstechens durchgeführt wurden.

10. Netzvorrichtung nach Anspruch 9, dadurch gekennzeichnet, daß:

- der Mikroprozessor (103) der Datenaufnahmestufe gesteuert wird durch das erste Anwendungsprogramm und ausgelegt ist, am Ende jedes Einstechens jede Rohdatenbasis an eine Datenverwaltungsstufe zu übertragen; wobei

- die Datenverwaltungsstufe einen Mikroprozessor (106), einen temporären Speicher (107) und einen permanenten Speicher (108) mit einem zweiten Auswerteprogramm und einem zweiten Anwendungsprogramm, Druckknöpfe (109) und eine Anzeigevorrichtung (110) zum Errichten einer interaktiven Verbindung zwischen der Datenverwaltungsstufe und dem Nutzer umfaßt;

- der Mikroprozessor (106) durch das zweite Anwendungsprogramm gesteuert wird und ausgelegt ist, um die folgenden Operationen durchzuführen:

    (1) in Abhängigkeit von Instruktionen des Benutzers, der zu diesem Zweck die Druckknöpfe (109) benutzt, auf der Anzeigevorrichtung (110) das Menü der unterschiedlichen möglichen Funktionen der Datenverwaltungsstufe anzuzeigen;

    (2) in Abhängigkeit von den Instruktionen des Nutzers die Ziffer des zu bearbeitenden Fleisches zu aktualisieren und diese Ziffer in dem temporären Speicher (107) abzuspeichern;

    (3) in dem temporären Speicher (107) die Rohdatenbasis zu speichern, die durch den Mikroprozessor (103) von der Datenaufnahmestufe übertragen wurde;

    (4) die Rohdatenbasis, die so gespeichert wurde, zu analysieren, um darin kennzeichnende Punkte zu erfassen, die zur Berechnung des Parameterwertes der Qualität des betreffenden Fleisches dienen;

(5) die Tiefen dieser kennzeichnenden Punkte in dem temporären Speicher (107) zu speichern;

(6) die Alarmfunktion (63) für den Fall anzusteuern, daß die fraglichen kennzeichnenden Punkte nicht in der analysierten Rohdatenbasis erfaßt werden konnten, wobei der Alarm den Nutzer informiert, daß das Einstechen nicht korrekt erfolgt ist und wiederholt werden muß;

(7) die Tiefen der kennzeichnenden Punkte, die in zwei Rohdatenbasen erfaßt wurden, die bei demselben Fleisch aufgenommen wurden, gemäß einer Gleichung zu kombinieren, die der Regel entspricht, die auf den gesuchten Qualitätsparameter anwendbar ist, der sich auf den Typ des gemessenen Fleisches bezieht, um einen numerischen Wert zu schaffen, der diesen Parameter widerspiegelt;

(8) diesen numerischen Wert in den temporären Speicher (107) abzuspeichern;

(9) den letzten numerischen Wert, der so berechnet wurde, auf der Anzeigevorrichtung (110) anzuzeigen;

(10) die Ziffer des Fleisches um eine Einheit zu erhöhen, die der nächsten Rohdatenbasis entspricht, die von der Datenaufnahmestufe zu empfangen ist;

(11) in Abhängigkeit von einer Instruktion des Nutzers an einen Zentralrechner den Inhalt des temporären Speichers (107) zu übertragen.

## Claims

1. Device for measuring the quality of the meat of a carcass, said device being of the type including :

   - a lancet-shaped probe (12) adapted to penetrate into the carcass and provided with an optical measuring head (18) ;
   - a reference depth support (20) associated with the probe (12) and intended to be applied to the carcass to be processed ;
   - a device (78-100) to continuously measure the distance separating the measuring head (18) from said support (20) ;
   - a measuring phototransmitter (36) and measuring photoreceiver (38) both constituting the measuring head (18) and disposed with respect to each other so as to produce an analog signal representative of the luminous reflectivity of the surrounding medium ;

   characterized in that :

   - the measuring phototransmitter (36) and measuring photoreceiver (38) are disposed at the bottom of fine optical channels (34b-32b) made in an opaque support (28), said channels being orientated so that their axes cross at a convergence point situated immediately outside the surface of the probe (12).

2. Measuring device according to claim 1, characterized in that an emission regulation photoreceiver (42) is disposed at the bottom of an optical channel (40b) opening into the fine channel (34b) of the phototransmitter (36).

3. Measuring device according to claim 1 or 2, characterized in that the optical transmission and receiving channels (34b-32b) are symmetrically slanted by an angle of about 12° with respect to a transverse plane of the rod (16) of the probe (12).

4. Measuring device according to any one of the preceding claims, characterized in that the optical channels (32b-34b) have a diameter of about 0.5 cm and are filled with a transparent resin.

5. Device for measuring the quality of the meat of a carcass according to any one of the preceding claims, characterized in that means for applying the support (20) to the carcass to be measured comprise a torque motor (94) automatically controlled so as to deliver a torque keeping to a set-point value the force applying the support (20) to the carcass.

6. Measuring device according to claim 5 and being of the type in which the probe (12) is a rigid hollow rod (16) mounted fixed in a housing (10), and the support (20), a mobile piston integral with two support rods (22-24) adapted to slide into the casing (10) and drive the device for measuring the driving-in depth of the measuring head (18), characterized in that the rod (16) of the probe (12) in its portion inside the casing (10) is embedded in two support blocks (60-62) respectively fixed to the ends of the base (64) of said casing, the support rods (22-24) of the piston (20) are mounted sliding in the front support (60) and embedded in a carriage (76) mounted sliding on said portion of the rod (16) inside the casing, said carriage (76) is integral with a synchronous belt (78) stretched between two pulleys (80-82) respectively mounted rotating in bearings borne by said support blocks (60-62), the shaft (81) of the pulley (80) comprising at one end a high resolution angular rotation optical sensor (98-100) delivering pulses to be counted up or down according to the direction of rotation and, at the other end, a first gear (90) gearing with a second gear (92) fixed to the shaft (81) of the torque motor (94).

7. Measuring device according to claim 5 and of the type in which the probe (12) is a rigid hollow rod (16) mounted fixed in a casing (10), and the support (20), a mobile piston integral with two support rods (22-24) adapted to slide into the casing (10) and drive the device for measuring the driving-in depth of the measuring head (18), characterized in that means (116-118-119) are provided to produce an analog signal representative of the rotation speed of the motor (94), in that a derivative stage (132) provided with a gain adjustment potentiometer (133) is adapted to deliver an analog signal representative of the angular acceleration of the motor (94), and in that a summing stage (128) is adapted to add a reference signal furnished by a potentiometer (130) and said acceleration signal, the adjustment of the gain potentiometer (133) being selected so that the variations of the support force of the piston (20) are compensated on a carcass due to the inertia of the mobile masses of the device during a measuring operation.

8. Measuring device according to claims 6 and 7, characterized in that :

   - the rotational speed signal of the motor (94) is applied to the input of a stage (134) for detecting the direction of rotation, said stage being adapted to deliver two logic signals S1-S2 representative of these directions when the absolute value of this speed is greater than a given threshold ;
   - a relatively wide flag (77) borne by the carriage (76) integral with the support rods (22-24) of the piston is adapted to hide two optical stop detectors (138-140) respectively mounted on the support blocks (60-62) of the rod (16) of the probe (12) and adapted to deliver two logic signals B1-B2 ;
   - a logic circuit (136) is adapted to receive the signals S1-S2 and B1-B2 and combine them so as to produce two logic signals C = S1+B1 and F = S1.B2 + S2.B1 ;
   - said logic signal C being control-applied to a potentiometric divider (123) disposed in series in a link established between the summing stage (128) and the current automatic control stage (120) ;
   - said logic signal F being control-applied for closing a switch (146) short-circuit disposed between the terminals of the motor (94).

9. Measuring device according to claims 2 and 6, characterized in that it comprises :

   - electronic circuits (37-41-43-45) to automatically control to a set value the amount of light emitted by the phototransmitter (36) received by the regulation photoreceiver (42) ;
   - an analog/digital converter (101) to deliver a digital signal representative of the light received by the measuring photoreceiver (38) and of the reflectivity of the layers traversed by the probe (12) ;
   - a reversible counter stage (102) to count up or down, according to the direction of rotation of the graduated dial (98) of an optical rotation sensor (100), the pulses delivered by this sensor and accordingly constantly provide the driving depth of the probe (12) into the carcass ;
   - a data acquisition stage including a microprocessor (103), a temporary memory (104) and a permanent memory (105) containing a first operating software and a first application software ;
   - said microprocessor (103) operating under the control of said first application software being adapted to :

     (1) periodically to call the contents of the output stages of the analog/digital converter (101) and the reversible counter (102) during the probe (12) withdrawal phase ;
     (2) combine the two numbers present at this moment in these output stages so as to produce two fields associated with measuring bits ;
     (3) apply these bit fields to a temporary memory (104) so as to progressively constitute a frame of raw data corresponding to the simultaneous light reflectivity and probe depth measurements carried out during a pricking operation.

10. Measuring device according to claim 9, characterized in that :

   - the microprocessor (103) of the data acquisition stage operating under the control of the first application software is adapted to transfer at the end of each pricking operation each frame of raw data to a data management stage ;
   - said data management stage including a microprocessor (106), a temporary memory (107) and a permanent memory (108) containing a second operating software and a second application software, push-buttons (109) and a display device (110) so as to establish an interactive link between said data management stage and the operator ;
   - said microprocessor (106) operating under the control of the second application software is adapted to carry out the following operations :

(1) in response to the instructions from the operator using to this effect the push-buttons (109), display on the display device (110) the menu of the various possible functions of the data management stage ;

(2) in response to the instructions from the operator, update the number of the carcass to be measured and store this number in the temporary memory (107) ;

(3) store in the temporary memory (107) the raw data frame transferred by the microprocessor (103) of the data acquisition stage ;

(4) analyse the stored raw data frame so as to detect therein the characteristic points to be retained for calculating the value of the meat quality parameter concerned ;

(5) store the depths of these characteristic points in the temporary memory (107) ;

(6) order the operation of an alarm (63) should the characteristic points in question were unable to be detected in the frame of the raw data analysed, said alarm informing the operator that the pricking operation carried out is incorrect and needs to be restarted ;

(7) combine the depths of the characteristic points detected in two frames of raw data read on a given carcass according to an equation conforming to the rules applicable to the sought-after quality parameter relating to the type of carcasses measured so as to produce a digital value representative of this parameter ;

(8) store this digital value in the temporary memory (107) ;

(9) display the latest digital value calculated on the display device (110) ;

(10) increment by one unit the carcass number corresponding to the next raw data frame to be received from the data acquisition stage ;

(11) in response to an instruction from the operator, transfer to a central computer the contents of the temporary memory (107).

FIG. 1

FIG. 5

FIG. 2

FIG. 3

EP 0 668 999 B1

FIG. 4

EP 0 668 999 B1

FIG. 6

45 REF

42 43 41
COMP.

36 37

38 39 101
C.A.N.

105 MEMOIRE PERMANENTE

103
MICRO-PROCESSEUR ⟹ (106)

80 POULIE  81  100 CAPTEUR OPTIQUE  102 COMPTEUR DECOMPTEUR

104 MEMOIRE TEMPORAIRE

108 MEMOIRE PERMANENTE

109 BOUTONS POUSSOIRS

106
MICRO-PROCESSEUR

(103) ⟹  ⟹ (79)

110 AFFICHAGE

107 MEMOIRE TEMPORAIRE

FIG. 7

FIG. 8

FIG. 9